# EUROPÄISCHE PATENTSCHRIFT

(11) **EP 3 899 421 B1**
(45) Veröffentlichungstag und Bekanntmachung des Hinweises auf die Patenterteilung: **07.02.2024**
(21) Anmeldenummer: 19835266.8
(22) Anmeldetag: 17.12.2019
(51) Int. Cl.: G01B 11/02, G01C 11/02

(54) **MESSSYSTEM ZUM MESSEN VON BEKLEIDUNGSSTÜCKEN**
MEASURING SYSTEM FOR MEASURING ARTICLES OF CLOTHING
SYSTÈME DE MESURE SERVANT À MESURER DES ARTICLES VESTIMENTAIRES

(30) Priorität: 20.12.2018 DE 102018133045
(43) Veröffentlichungstag der Anmeldung: 27.10.2021
(73) Patentinhaber: Droste, Sascha, 48599 Gronau (DE)
(72) Erfinder: Droste, Sascha, 48599 Gronau (DE)
(74) Vertreter: AWA Denmark A/S
(86) Internationale Anmeldenummer: PCT/EP2019/085587
(87) Internationale Veröffentlichungsnummer: WO 2020/127219

(56) Entgegenhaltungen:
- US-A1- 2014 059 981
- US-A1- 2016 117 749
- US-A1- 2017 303 616

## Beschreibung

Die Erfindung betrifft ein Messsystem zum Messen von Bekleidungsstücken, umfassend einen Messtisch mit einer Tischoberfläche, auf die die Bekleidungsstücke gelegt und dann vermessen werden.

### HINTERGRUND DER ERFINDUNG

In der Bekleidungsindustrie ist es üblich, die Bekleidungsstücke nach der Fertigung in Bezug auf Qualität und Genauigkeit der Abmessungen zu kontrollieren. Hierzu werden die Bekleidungsstücke von einer Kontrolleurin / einem Kontrolleur auf einen Messtisch gelegt und optisch kontrolliert. Außerdem werden verschiedene Abmessungen, wie z. B. die Bundweite, Hüftweite oder die Schrittlänge einer Hose mittels eines Maßbands von Hand nachgemessen. Die einzelnen Messdaten werden notiert und können dann mit den für das Bekleidungsstück vorgegebenen Sollwerten verglichen werden. Ein solches manuelles Verfahren ist jedoch relativ kompliziert und zeitaufwändig.

Dokument US2016/117749A1 offenbart ein Verfahren und ein System zur Empfehlung von taillierter Kleidung. Dokument US2014/059981A1 offentbart einen Apparat, Systeme und Verfahren zur Anwendung eines Handhelden Messgerät zum Erzeugen von On-Demand-Verpackung.

### AUFGABE DER ERFINDUNG

Es ist daher eine Aufgabe der vorliegenden Erfindung, ein Messsystem zum Messen von Bekleidungsstücken zu schaffen, welches die gesamte Messprozedur einfacher und schneller macht.

Gelöst wird diese Aufgabe gemäß der Erfindung durch die im unabhängigen Anspruch 1 genannten Merkmale. Weitere Ausführungsformen der Erfindung ergeben sich aus den Unteransprüchen.

Gemäß der Erfindung wird ein Messsystem zum Messen von Bekleidungsstücken vorgeschlagen, das wenigstens folgende Komponenten umfasst:
- einen Messtisch mit einer Tischoberfläche, auf die das zu messende Bekleidungsstück aufgelegt wird,
- wenigstens einen Laser-Entfernungsmesser, der einen Laserstrahl aussendet, wobei der Laser-Entfernungsmesser so angeordnet ist, dass der Laserstrahl in einem geringen Abstand parallel zur Tischoberfläche verläuft, so dass er, wenn ein Bekleidungsstück auf dem Messtisch liegt, seitlich auf das Bekleidungsstück trifft,
- wenigstens eine Markierung, an der das Bekleidungsstück angelegt wird, bevor eine Messung mittels des wenigstens einen Laser-Entfernungsmessers durchgeführt wird, und
- eine Datenverarbeitungseinrichtung, die unter Berücksichtigung der Position der wenigstens einen Markierung und eines von dem oder den Laser-Entfernungsmessern gemessenen Wert ein bestimmtes Längenmaß des Bekleidungsstücks ermittelt. Dies hat den Vorteil, dass die Längenmessung wesentlichen schneller und einfacher durchgeführt werden kann.

Bei der Datenverarbeitungseinrichtung kann es sich z. B. um einen herkömmlichen PC handeln. Als Laser-Entfernungsmesser kann beispielsweise ein Analogsensor der Serie HG-C von Panasonic genutzt werden.

Gemäß einer bevorzugten Ausführungsform der Erfindung umfasst das Messsystem mehrere Laser-Entfernungsmesser. In diesem Fall sind vorzugsweise wenigstens zwei der Laser-Entfernungsmesser auf unterschiedlichen Seiten des Bekleidungsstücks bzw. eines Bereichs der Tischoberfläche, auf den das zu messende Bekleidungsstück aufgelegt wird, angeordnet.

Die vorstehend genannte wenigstens eine Markierung wird vorzugsweise mittels einer Lichtquelle, insbesondere mittels einer Laserlichtquelle erzeugt. Die Lichtquelle ist vorzugsweise oberhalb des Messtisches angeordnet und projiziert die Markierung, wie z. B. einen Lichtpunkt, auf den Messtisch bzw. das Bekleidungsstück. Alternativ kann aber auch eine Markierung, wie z. B. ein farbiger Punkt oder Strich, auf der Tischoberfläche aufgebracht sein.

Auf der Tischoberfläche können auch mehrere Hilfslinien angeordnet sein, die eine Kontrolleurin bzw. einen Kontrolleur beim Ausrichten der zu messenden Bekleidungsstücke auf der Tischoberfläche unterstützen.

Der bzw. die Laser-Entfernungsmesser sind vorzugsweise auf der Tischoberfläche des Messtisches montiert.

Gemäß einer bevorzugten Ausführungsform der Erfindung umfasst das Messsystem ferner einen Scanner, mit dem das zu messende Bekleidungsstück eingescannt werden kann. Unter dem Begriff "einscannen" ist dabei zu verstehen, dass Informationen, wie z. B. ein Barcode oder andere auf einem Etikett enthaltene Angaben, eingelesen werden, aus denen sich die Soll-Abmessungen des betreffenden Bekleidungsstücks ermitteln lassen. Dadurch wird es möglich, die Ist-Werte mit den Soll-Werten des speziellen Bekleidungsstücks zu vergleichen.

Das erfindungsgemäße Messsystem kann ferner einen Bildschirm umfassen, an dem das Messergebnis angezeigt wird. Zusätzlich können auch die Soll-Abmessungen des Bekleidungsstücks oder eine Abweichung zwischen den Ist- und Soll-Werten angezeigt werden.

Bei einer Ausführungsform mit mehreren Laser-Entfernungsmessern sind vorzugsweise wenigstens zwei der Laser-Entfernungsmesser in einem Winkel zueinander angeordnet, so dass sich die von den beiden Laser-Entfernungsmessern ausgesendeten Laserstrahlen in einem Bereich der Tischoberfläche, auf den das zu messende Bekleidungsstück aufgelegt wird, kreuzen (zumindest, wenn sie auf gleicher Höhe über dem Tisch verlaufen). Mittels der im Winkel angeordneten Laser-Entfernungsmesser kann beispielsweise die Hüftweite einer Hose gut gemessen werden.

Gemäß einer bevorzugten Ausführungsform der Erfindung umfasst das Messsystem genau drei Laser-Entfernungsmesser.

### KURZE BESCHREIBUNG DER ZEICHNUNGEN

Die Erfindung wird nachstehend anhand der beigefügten Zeichnungen beispielhaft näher erläutert. Es zeigen:
Fig. 1 eine Aufsicht auf ein Messsystem zum Abmessen von Bekleidungsstücken gemäß einer Ausführungsform der Erfindung;
Fig. 2 eine Aufsicht auf das Messsystem von Fig. 1, wobei das Messsystem für die Messung der Bundweite einer Hose genutzt wird;
Fig. 3 eine Aufsicht auf das Messsystem von Fig. 1, wobei das Messsystem für die Messung der Hüftweite einer Hose genutzt wird; und
Fig. 4 eine Aufsicht auf das Messsystem von Fig. 1, wobei das Messsystem für die Messung der Schrittlänge einer Hose genutzt wird.

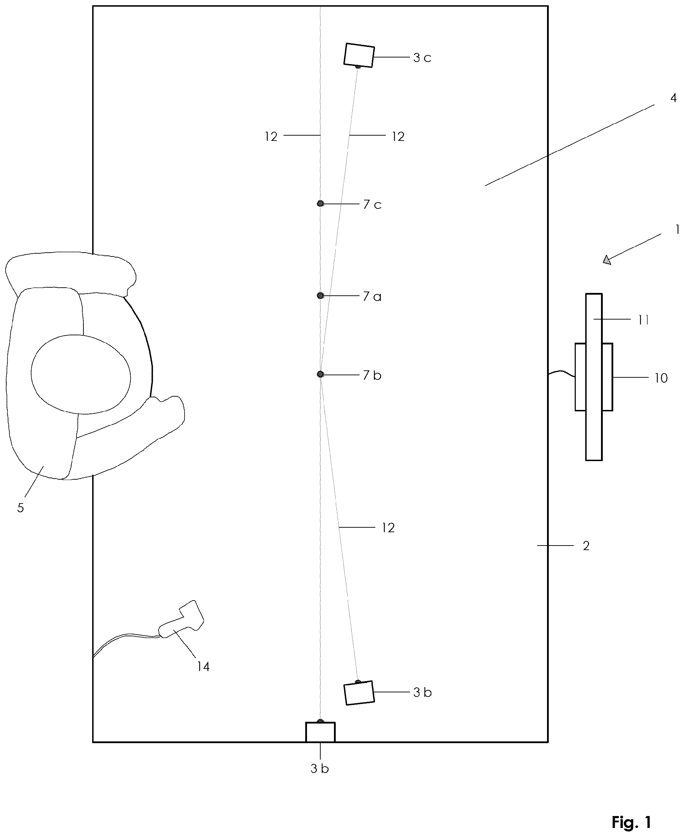

Fig. 1 zeigt ein Messsystem 1 zum Messen von Bekleidungsstücken 6, mit dem eine Kontrolleurin / ein Kontrolleur die Abmessungen eines produzierten Bekleidungsstücks 6 überprüfen kann. Das Messsystem 1 umfasst einen Messtisch 2 mit einer Tischoberfläche 4, auf die das zu überprüfende Bekleidungsstück 6 aufgelegt wird. Auf der Tischoberfläche 4 sind mehrere - hier drei - Laser-Entfernungsmesser 3a - 3c angeordnet, die jeweils einen Laserstrahl 8a - 8c aussenden, der in einem geringen Abstand (z. B. 0,3 cm bis 0,8 cm) parallel zur Tischoberfläche 4 verläuft, so dass er, wenn ein Bekleidungsstück 6 auf dem Messtisch 2 angeordnet ist, von der Seite auf das Bekleidungsstück 6 trifft.

Die einzelnen Laser-Entfernungsmesser 3a - 3c sind so auf der Tischoberfläche 4 des Messtisches 2 angeordnet, dass verschiedene Abmessungen eines Bekleidungsstücks, wie z. B. die Bundweite, die Hüftweite oder die Schrittlänge genau gemessen werden können. Für die Messung gerader Abmessungen, wie z. B. der Bundweite oder der Schrittlänge einer Hose ist nur ein einziger Laser-Entfernungsmesser - hier der Laser-Entfernungsmesser 3a - erforderlich. Zur Messung der Hüftweite sind hier die Laser-Entfernungsmesser 3b und 3c vorgesehen. Die beiden letztgenannten Laser-Entfernungsmesser 3b, 3c sind in einem Winkel zueinander angeordnet und senden jeweils einen Laserstrahl 8b, 8c aus, die sich in einem Bereich der Tischoberfläche 4, auf den das zu überprüfende Bekleidungsstück 6 aufgelegt wird, kreuzen.

Je nach Art der Messung sind ein oder mehrere der Laser-Entfernungsmesser 3a - 3c aktiv geschaltet. Der bzw. die nicht benötigten Laser- Entfernungsmesser 3a - 3c sind dann vorzugsweise deaktiviert.

Auf der Tischoberfläche 4 sind ferner mehrere Hilfslinien 12 angeordnet, die der Kontrolleurin / dem Kontrolleur beim richtigen Ausrichten der zu überprüfenden Bekleidungsstücke 6 helfen.

Auf der Tischoberfläche 4 des Messtisches 2 von Fig. 1 sind außerdem mehrere Markierungen 7a - 7c zu erkennen, die der Kontrolleurin / dem Kontrolleur 5 als Referenzpunkt dienen und ihr/ihm anzeigen, wo das Bekleidungsstück 6 angelegt werden muss, bevor eine Lasermessung durchgeführt wird. Im dargestellten Ausführungsbeispiel sind drei Markierungen 7a - 7c vorgesehen, die als Anlegepunkte für unterschiedliche Messungen dienen.

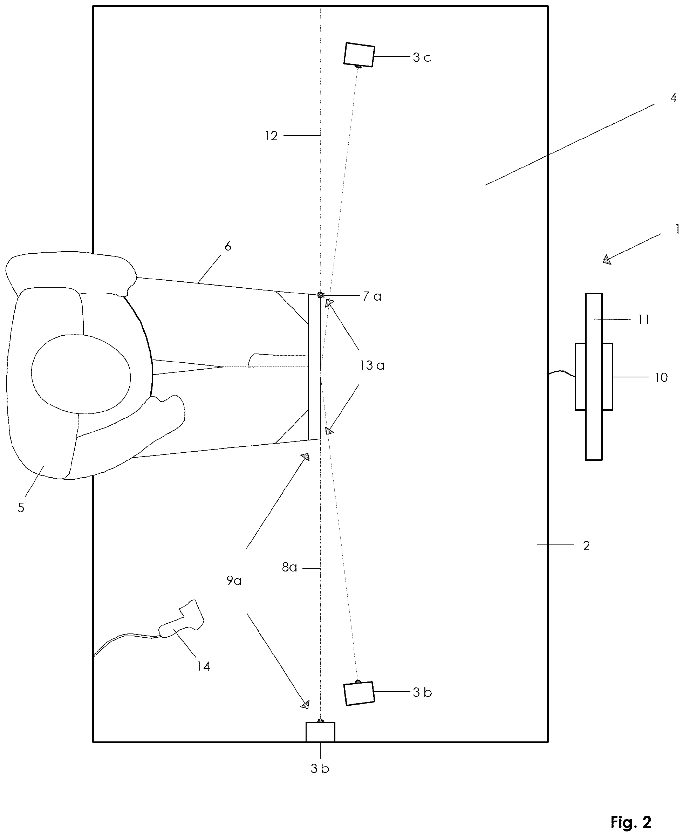

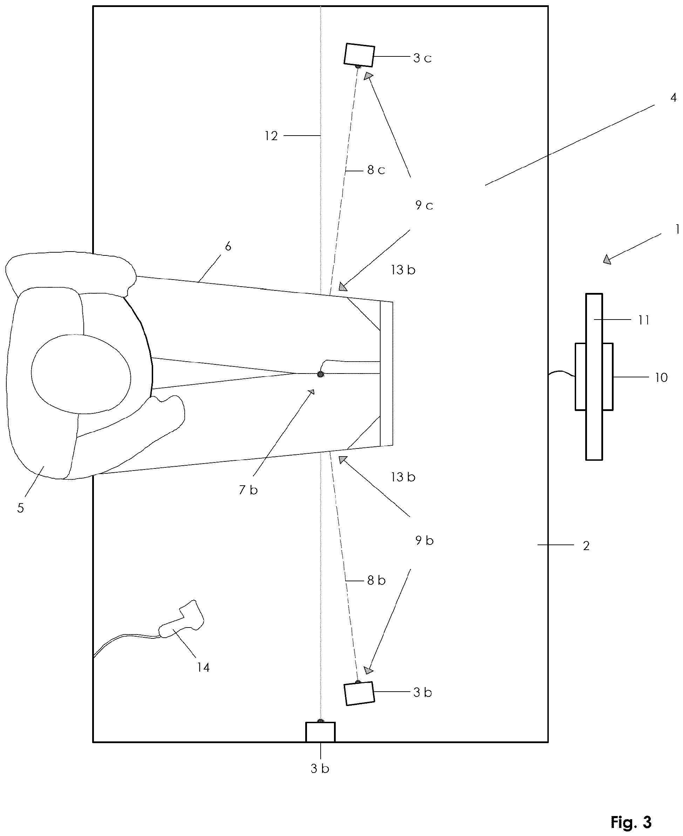

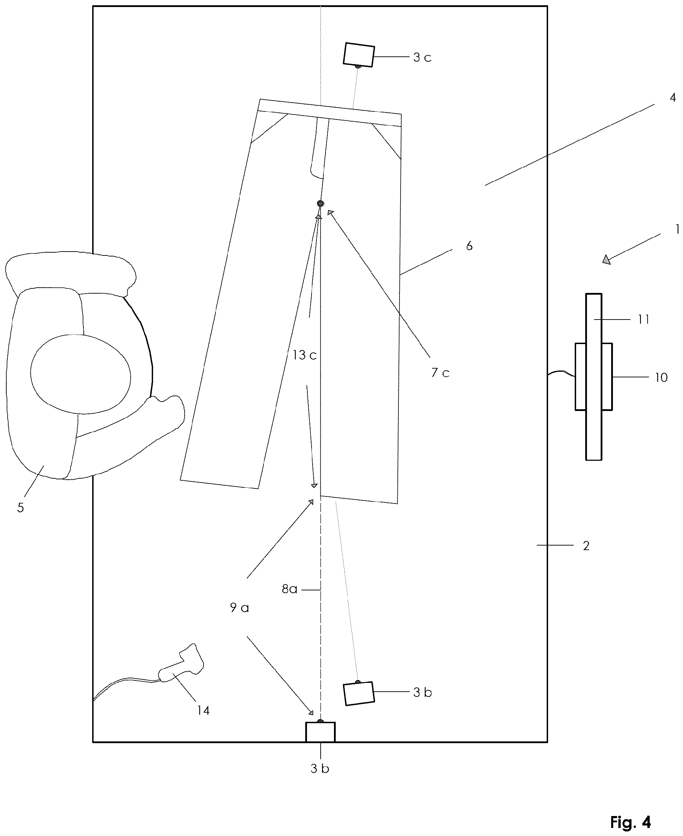

Im dargestellten Ausführungsbeispiel markiert die Markierung 7a einen Anlegepunkt, an dem die Kontrolleurin/der Kontrolleur 5 beispielsweise den Bund einer Hose seitlich anlegen muss, um die Bundweite 13a zu messen, wie in Fig. 2 gezeigt ist. Die Markierung 7b dient dazu, der Kontrolleurin/dem Kontrolleur 5 anzuzeigen, wo sie/er beispielsweise eine Kreuznaht einer Hose anlegen muss, um die Hüftweite 13b einer Hose zu messen, wie in Fig. 3 dargestellt ist, und die Markierung 7c dient dazu, der Kontrolleurin/dem Kontrolleur 5 anzuzeigen, wo sie/er die Kreuznaht einer Hose anlegen muss, um beispielsweise die Schrittlänge 13c einer Hose zu messen, wie in Fig. 4 dargestellt ist.

Die einzelnen Markierungen 7a - 7c werden vorzugsweise durch jeweils eine oberhalb des Messtisches 2 angeordnete Lichtquelle (nicht gezeigt), insbesondere eine Laserlichtquelle, erzeugt. Je nach Art der Messung ist vorzugsweise nur eine der Markierungen 7a - 7c sichtbar. Die übrigen Lichtquellen sind vorzugsweise inaktiv.

Das in Fig. 1 dargestellte Messsystem umfasst vorzugsweise auch einen Scanner 14, mit dem das zu überprüfende Bekleidungsstück 6 gescannt werden kann. Als Scanner kann beispielsweise eine Barcodelaser oder ein anderer optischer Scanner eingesetzt werden, der in der Lage ist, einen Code oder andere optische Merkmale eines zu überprüfenden Bekleidungsstücks 6 zu erkennen und daraus die zugehörigen Soll-Abmessungen des betreffenden Bekleidungsstücks 6 wie z. B. eine Soll-Bundweite, eine Soll-Hüftweite oder eine Soll-Schrittlänge zu ermitteln. Gemäß einer speziellen Ausführungsform der Erfindung kann beispielsweise ein Scanner 14 vorgesehen sein, der in der Lage ist, ein am Bekleidungsstück 6 befestigtes Etikett zu lesen und darauf mittels Schrifterkennung beispielsweise den Namen des Herstellers und eine Größenangabe zu erkennen.

Das Messsystem umfasst ferner eine Datenverarbeitungseinrichtung 10, die mit den Laser-Entfernungsmessern 3a - 3c, dem Scanner 14 und/oder den Lichtquellen (nicht gezeigt) in Verbindung steht und die einzelnen Komponenten 3a - 3c, 14 ansteuert sowie die von den Komponenten 3a - 3c, 14 gelieferten Daten verarbeitet. Außerdem ist ein Bildschirm 11 vorgesehen, an dem das jeweilige Messergebnis angezeigt wird.

Die Funktionsweise des in Fig. 1 dargestellten Messsystems wird im Folgenden anhand verschiedener Beispiele näher erläutert.

Fig. 2 zeigt das Messsystem 1 von Fig. 1, wobei die Bundweite 13a einer Hose gemessen wird. In einem ersten Schritt scannt die Kontrolleurin / der Kontrolleur 5 zunächst mit Hilfe des Scanners 14 die Hose ein. Die gescannten Daten werden dann an die Datenverarbeitungseinrichtung 10 übermittelt, die daraus wiederum die Soll-Abmessungen des Bekleidungsstücks 6, im vorliegenden Fall beispielsweise die Soll-Bundweite, die Soll-Hüftweite und die Soll-Schrittlänge der zu überprüfenden Hose ermittelt. Die Soll-Werte sind in einer Datenbank hinterlegt.

Da im vorliegenden Fall die Bundweite 13a der Hose gemessen wird, wird nur die Markierung 7a, die einen Anlegepunkt für den Bund der Hose markiert, angezeigt. Die Kontrolleurin / der Kontrolleur 5 kann nun die Hose in der vorgeschriebenen Weise (wie dargestellt) an der Markierung 7a anlegen. Die auf der Tischoberfläche 4 angezeigten Hilfslinien 12 unterstützen sie/ihn dabei beim Ausrichten der Hose auf dem Messtisch 2.

Sobald die Hose in der richtigen Position liegt, drückt die Kontrolleurin/der Kontrolleur 5 ein Fußpedal oder einen Knopf (nicht gezeigt), wodurch mittels des Laser-Entfernungsmessers 3a eine Messung der Bundweite durchgeführt wird. Der Laser-Entfernungsmesser 3a misst in diesem Fall eine Messstrecke 9a zwischen dem Laser-Entfernungsmesser 3a und dem Bund der Hose. Da die Position der Markierung 7a bekannt ist, kann die Bundweite 13a unter Berücksichtigung der Position der Markierung 7a und der Messstrecke 9a bestimmt werden. Die Bestimmung der Bundweite 13a erfolgt vorzugsweise in der Datenverarbeitungseinrichtung 10. Das Messergebnis und ggfs. auch eine Abweichung von der Soll-Bundweite kann ggfs. auf einem Bildschirm 11 angezeigt werden. Das Messergebnis wird vorzugsweise gespeichert und kann zu einem späteren Zeitpunkt wieder aufgerufen werden.

Bei der Messung der Bundweite 13a ist vorzugsweise nur der Laser-Entfernungsmesser 3a aktiv. Die beiden anderen Laser-Entfernungsmesser 3b, 3c sind vorzugsweise deaktiviert.

Fig. 3 zeigt eine Ansicht des Messsystems 1 von Fig. 1, das in diesem Fall dazu genutzt wird, die Hüftweite 13b einer Hose zu messen.

Bei der Hüftweitenmessung wird nur die Markierung 7b auf den Messtisch 2 projiziert. Die beiden anderen Lichtquellen werden vorzugsweise ausgeschaltet. Die Markierung 7b zeigt der Kontrolleurin / dem Kontrolleur 5 an, wo sie/er eine Kreuznaht der Hose anlegen soll. Die Hilfslinien 12 unterstützen sie wiederum beim Ausrichten der Hose.

Durch Drücken des Fußpedals (nicht gezeigt) wird wiederum eine Messung ausgelöst - diesmal allerdings mittels der beiden Laser-Entfernungsmesser 3b und 3c. Der Laser-Entfernungsmesser 3a ist ausgeschaltet. Jeder der Laser-Entfernungsmesser 3b, 3c misst jeweils eine Messstrecke 9b, 9c zwischen dem betreffenden Entfernungsmesser und der Hüfte der Hose. Nachdem die Position der Markierung 7b wiederum bekannt ist, kann die Hüftweite 13b der Hose unter Berücksichtigung dieser Position und der beiden Messstrecken 9b, 9c berechnet werden. Die Bestimmung der Hüftweite 13b erfolgt vorzugsweise in der Datenverarbeitungseinrichtung 10. Das Messergebnis und ggfs. eine Abweichung von der Soll-Hüftweite kann beispielsweise auf einem Bildschirm 11 angezeigt werden.

Fig. 4 zeigt schließlich noch das Messsystem 1 von Fig. 1, das in diesem Fall zum Messen der Schrittlänge 13c einer Hose genutzt wird. Bei der Schrittlängenmessung wird nur die Markierung 7c angezeigt. Die beiden anderen Lichtquellen sind ausgeschaltet. Die Kontrolleurin / der Kontrolleur 5 kann nun die Hose in der vorgegebenen Weise an der Markierung 7c anlegen und das zu überprüfende Hosenbein an der Hilfslinie 12 ausrichten, wie in Fig. 4 dargestellt ist.

Zum Durchführen der Messung drückt die Kontrolleurin / der Kontrolleur 5 wiederum das Fußpedal. In diesem Fall wird die Messung ausschließlich vom Laser-Entfernungsmesser 3a ausgeführt, der eine Messstrecke 9a zwischen dem Laser-Entfernungsmesser 3a und dem Ende des Hosenbeins misst. Da nun die Position der Markierung 7c und die Messstrecke 9a bekannt sind, kann unter Berücksichtigung dieser Werte wiederum die Schrittlänge 13c der Hose berechnet werden.

Das Messsystem ist vorzugsweise derart eingerichtet, dass nach jeder Messung automatisch die passende Konfiguration für die nachfolgende Messung eingestellt wird. Nach der Messung der Bundweite wird das Messsystem 1 beispielsweise automatisch so konfiguriert, dass in einer nächsten Messung die Hüftweise und in einer darauffolgenden Messung dann die Schrittlänge einer Hose gemessen werden kann. In diesem Zusammenhang wird beispielsweise definiert, welcher Laser-Entfernungsmesser 3a - 3c aktiv oder passiv geschaltet wird, welche Lichtquelle zur Erzeugung einer Markierung 7a - 7c ausgewählt wird oder welche Daten an die DV-Einrichtung übertragen und verarbeitet werden.

Die Anzahl, Art und Reihenfolge der einzelnen Messungen kann im Prinzip nach Wunsch definiert werden. Die Steuerung der gesamten Messprozedur erfolgt vorzugsweise mittels der Datenverarbeitungseinrichtung 10. Es kann aber auch eine andere Steuereinheit vorgesehen sein.

## Patentansprüche

1. Messsystem zum Messen von Bekleidungsstücken (6), umfassend:
- einen Messtisch (2) mit einer Tischoberfläche (4), auf die das zu messende Bekleidungsstück (6) aufgelegt wird,
- wenigstens einen Laser-Entfernungsmesser (3a - 3c), der einen Laserstrahl (8a - 8c) aussendet, wobei der Laser-Entfernungsmesser (3a - 3c) so angeordnet ist, dass der Laserstrahl (8a - 8c) in einem geringen Abstand parallel zur Tischoberfläche (4) verläuft, so dass er, wenn ein Bekleidungsstück (6) auf dem Messtisch (2) liegt, auf das Bekleidungsstück (6) trifft,
- wenigstens eine oberhalb des Messtisches angeordnete Lichtquelle, mittels der wenigstens eine auf der Tischoberfläche des Messtisches erkennbare Markierung (7a - 7c) erzeugt wird, an der das Bekleidungsstück (6) angelegt wird, bevor eine Messung mittels des wenigstens einen Laser-Entfernungsmessers (3a - 3c) durchgeführt wird, und
- eine Datenverarbeitungseinrichtung (10), die unter Berücksichtigung der Position der wenigstens einer Markierung und eines von dem oder den Laser-Entfernungsmessern (3a - 3c) gemessenen Werts ein Längenmaß des Bekleidungsstücks (6) ermittelt.

2. Messsystem nach Anspruch 1,
**dadurch gekennzeichnet, dass**
es mehrere Laser-Entfernungsmesser (3a - 3c) umfasst.

3. Messsystem nach Anspruch 2,
**dadurch gekennzeichnet, dass**
wenigstens zwei der Laser-Entfernungsmesser (3a - 3c) auf unterschiedlichen Seiten eines Bereichs der Tischoberfläche (4), auf den das zu messende Bekleidungsstück (6) aufgelegt wird, angeordnet sind.

4. Messsystem nach einem der vorhergehenden Ansprüche,
**dadurch gekennzeichnet, dass**
die Lichtquelle eine Laserlichtquelle ist.

5. Messsystem nach einem der vorhergehenden Ansprüche,
**dadurch gekennzeichnet, dass**
auf der Tischoberfläche (4) mehrere Hilfslinien (12) angeordnet sind, die eine Kontrolleurin bzw. einen Kontrolleur beim Ausrichten der Bekleidungsstücke (6) auf der Tischoberfläche (4) unterstützen.

6. Messsystem nach einem der vorhergehenden Ansprüche,
**dadurch gekennzeichnet, dass**
der wenigstens eine Laser-Entfernungsmesser (3a - 3c) auf der Tischoberfläche (4) des Messtisches (2) montiert ist.

7. Messsystem nach einem der vorhergehenden Ansprüche,
**dadurch gekennzeichnet, dass**
es ferner einen Bildschirm (11) umfasst, an dem das Messergebnis der mittels des wenigstens einen Laser-Entfernungsmessers (3a - 3c) durchgeführten Messung angezeigt wird.

8. Messsystem nach einem der vorhergehenden Ansprüche,
**dadurch gekennzeichnet, dass**
es mehrere Laser-Entfernungsmesser (3a - 3c) umfasst, wobei wenigstens zwei der Laser-Entfernungsmesser (3a - 3c) in einem Winkel zueinander angeordnet sind, so dass sich die von den beiden Laser-Entfernungsmessern (3a - 3c) ausgesendeten Laserstrahlen (8a - 8c) in einem Bereich der Tischoberfläche (4), auf den das zu messende Bekleidungsstück (6) aufgelegt wird, kreuzen.

9. Messsystem nach einem der vorhergehenden Ansprüche,
**dadurch gekennzeichnet, dass**
es genau drei Laser-Entfernungsmesser (3a - 3c) umfasst.

## Claims

1. Measuring system for measuring articles of clothing (6), comprising:
- a measuring table (2) having a table surface (4), the article of clothing (6) to be measured being placed onto said table surface,
- at least one laser distance measuring device (3a-3c) which emits a laser beam (8a-8c), wherein the laser distance measuring device (3a-3c) is arranged such that the laser beam (8a-8c) passes parallel to the table surface (4) at a small distance so that, when an article of clothing (6) is on the measuring table (2), said laser beam impinges on the article of clothing (6),
- at least one light source which is arranged above the measuring table and which is used to generate at least one marking (7a-7c) which is discernible on the table surface of the measuring table and on which the article of clothing (6) is placed before a measurement is carried out by means of the at least one laser distance measuring device (3a-3c), and
- a data processing device (10), which determines a length dimension of the article of clothing (6) taking account of the position of the at least one marking and a value measured by the one or more laser distance measuring devices (3a-3c).

2. Measuring system according to Claim 1,
**characterized in that**
it comprises a plurality of laser distance measuring devices (3a-3c).

3. Measuring system according to Claim 2,
**characterized in that**
at least two of the laser distance measuring devices (3a-3c) are arranged on different sides of a region of the table surface (4) onto which the article of clothing (6) to be measured is placed.

4. Measuring system according to any of the preceding claims,
**characterized in that**
the light source is a laser light source.

5. Measuring system according to any of the preceding claims,
**characterized in that**
a plurality of auxiliary lines (12) are arranged on the table surface (4) and assist an inspector when orienting the articles of clothing (6) on the table surface (4).

6. Measuring system according to any of the preceding claims,
**characterized in that**
the at least one laser distance measuring device (3a-3c) is mounted on the table surface (4) of the measuring table (2).

7. Measuring system according to any of the preceding claims,
**characterized in that**
it furthermore comprises a screen (11), on which the measurement result of the measurement carried out by means of the at least one laser distance measuring device (3a-3c) is displayed.

8. Measuring system according to any of the preceding claims,
**characterized in that**
it comprises a plurality of laser distance measuring devices (3a-3c), wherein at least two of the laser distance measuring devices (3a-3c) are arranged at an angle with respect to one another, such that the laser beams (8a-8c) emitted by the two laser distance measuring devices (3a-3c) intersect in a region of the table surface (4) onto which the article of clothing (6) to be measured is placed.

9. Measuring system according to any of the preceding claims,
**characterized in that**
it comprises exactly three laser distance measuring devices (3a-3c).

## Revendications

1. Système de mesure servant à mesurer des articles vestimentaires (6), comprenant :
- une table de mesure (2) pourvue d'une surface de table (4), sur laquelle est placé l'article vestimentaire (6) à mesurer,
- au moins un télémètre laser (3a - 3c), qui émet un faisceau laser (8a - 8c), dans lequel le télémètre laser (3a - 3c) est disposé de telle sorte que le faisceau laser (8a - 8c) s'étende parallèlement et à faible distance de la surface de table (4), de manière à ce que, lorsqu'un article vestimentaire (6) est placé sur la table de mesure (2), il atteigne l'article vestimentaire (6),
- au moins une source de lumière disposée au-dessus de la table de mesure, au moyen de laquelle est généré au moins un repère (7a - 7c) reconnaissable sur la surface de table de la table de mesure, sur lequel l'article vestimentaire (6) est appliqué avant qu'une mesure ne soit effectuée au moyen dudit au moins un télémètre laser (3a - 3c), et
- un dispositif de traitement de données (10), qui détermine une cote longitudinale de l'article vestimentaire (6) en tenant compte de la position dudit au moins un repère et d'une valeur mesurée par le ou les télémètres laser (3a - 3c).

2. Système de mesure selon la revendication 1, **caractérisé en ce qu'**il comprend plusieurs télémètres laser (3a - 3c).

3. Système de mesure selon la revendication 2, **caractérisé en ce qu'**au moins deux des télémètres laser (3a - 3c) sont disposés sur des côtés différents d'une zone de la surface de table (4) sur laquelle est placé l'article vestimentaire (6) à mesurer.

4. Système de mesure selon l'une des revendications précédentes,
**caractérisé en ce que** la source de lumière est une source de lumière laser.

5. Système de mesure selon l'une des revendications précédentes,
**caractérisé en ce que** plusieurs lignes auxiliaires (12) sont disposées sur la surface de table (4), pour aider une contrôleuse ou un contrôleur à aligner les articles vestimentaires (6) sur la surface de table (4).

6. Système de mesure selon l'une des revendications précédentes,
**caractérisé en ce que** ledit au moins un télémètres laser (3a - 3c) est monté sur la surface de table (4) de la table de mesure (2).

7. Système de mesure selon l'une des revendications précédentes,
**caractérisé en ce qu'**il comprend en outre un écran (11) sur lequel est affiché le résultat de mesure de la mesure effectuée au moyen dudit au moins un télémètre laser (3a - 3c).

8. Système de mesure selon l'une des revendications précédentes,
**caractérisé en ce qu'**il comprend plusieurs télémètres laser (3a-3c), dans lequel au moins deux des télémètres laser (3a - 3c) sont disposés selon un certain angle l'un par rapport à l'autre, de sorte que les faisceaux laser (8a - 8c) émis par les deux télémètres laser (3a - 3c) se croisent dans une zone de la surface de table (4) sur laquelle est placé l'article vestimentaire (6) à mesurer.

9. Système de mesure selon l'une des revendications précédentes,
**caractérisé en ce qu'**il comprend exactement trois télémètres laser (3a - 3c).
